(19) Europäisches Patentamt
European Patent Office
Office européen des brevets

(11) **EP 4 322 043 A1**

(12) **EUROPEAN PATENT APPLICATION**

(43) Date of publication:
**14.02.2024 Bulletin 2024/07**

(21) Application number: **23181741.2**

(22) Date of filing: **27.06.2023**

(51) International Patent Classification (IPC):
**G06F 30/20** (2020.01)   **G06F 30/25** (2020.01)
**G06F 30/28** (2020.01)   **H01M 10/056** (2010.01)
**G16C 10/00** (2019.01)   **G16C 20/20** (2019.01)
**G16C 20/30** (2019.01)

(52) Cooperative Patent Classification (CPC):
**H01M 10/056; G06F 30/20; G06F 30/25;
G06F 30/28; G16C 10/00; G16C 20/20;
G16C 20/30;** H01M 2300/00; Y02E 60/10

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **28.06.2022 IN 202221037132**

(71) Applicant: **Tata Consultancy Services Limited
Maharashtra (IN)**

(72) Inventors:
• **GARAPATI, Vamsi Krishna
411013 Pune, Maharashtra (IN)**

• **BADWEKAR, Kaustubh Rajendra
411013 Pune, Maharashtra (IN)**
• **NAGA NEEHAR, Dingari
411013 Pune, Maharashtra (IN)**
• **MYNAM, Mahesh
411013 Pune, Maharashtra (IN)**
• **RAI, Beena
411013 Pune, Maharashtra (IN)**

(74) Representative: **Goddar, Heinz J.
Boehmert & Boehmert
Anwaltspartnerschaft mbB
Pettenkoferstrasse 22
80336 München (DE)**

(54) **METHOD AND SYSTEM FOR IDENTIFYING ELECTROLYTE COMPOSITION FOR OPTIMAL BATTERY PERFORMANCE**

(57)    The present invention relates to the field of electrolyte design. Existing methods focus on optimizing the electrolyte composition on a stand-alone basis with respect to its properties and validating battery performance experimentally which is a time-consuming process. Thus, embodiments of present disclosure provide an automated method and system for identifying electrolyte composition for optimal battery performance. The system receives certain input parameters and computes transport properties using the input. Then, a feasible electrolyte composition is identified from a material database based on deviation index metric. The identified electrolyte composition is then optimized based on the input by considering the deviation index and battery performance metrics such as capacity fade and internal heat generation. Simulation case studies performed show that the method is capable of identifying a new electrolyte from the material database as well as identify optimal concentration of same electrolyte which results in better performance of the battery.

FIG. 3

EP 4 322 043 A1

**Description**

CROSS-REFERENCE TO RELATED APPLICATIONS AND PRIORITY

The present application claims priority to Indian application no. 202221037132, filed on June 28, 2022.

TECHNICAL FIELD

The present invention generally relates to the field of electrolyte design and, more particularly, to method and system for identifying electrolyte composition for optimal battery performance.

BACKGROUND

Batteries play a significant role in reducing the usage of non-renewable energy sources such as fossil fuels for transportation. Currently available batteries fall short to meet the immediate demands such as high energy density, longer cycle life, fast charging capability etc., and efforts have been focused towards finding novel materials to address the performance issues. The discovery of new electrolytes with desired properties plays a pivotal role in the development of next generation batteries. Traditional way of design and optimization of electrolyte involves a) experimental characterization of the transport properties of the electrolyte, or/and b) experimental characterization of the influence of new electrolyte material on the battery performance. This process is expensive and involves a lot of trial and error. Also, simulations at different length-scales are required to obtain transport properties of the electrolyte and study influence of transport properties on battery performance. It is challenging to combine simulations at different length scales due to variation in the computational times. In addition, it is difficult to cover the entire sample space of possible electrolyte candidates using only experiments.

Over the last few years, researchers have worked on Machine Learning (ML) and Artificial Intelligence (AI) based screening of novel electrolyte materials based on their transport properties (ionic conductivity/diffusivity). ML based methods have shown to aid high-throughput screening of feasible salt or solvent candidates, thereby reducing the development time. AI driven approaches speed up the discovery of salts and solvents. However, the ML and AI driven approaches rest on the individual properties of electrolyte components and fail to address the influence of electrolyte on the battery performance.

SUMMARY

Embodiments of the present disclosure present technological improvements as solutions to one or more of the above-mentioned technical problems recognized by the inventors in conventional systems. For example, in one embodiment, a method for identifying electrolyte composition for optimal battery performance is provided. The method comprises receiving (i) one or more initial material parameters, (ii) one or more physics-based model parameters, (iii) one or more optimization parameters, and (iv) one or more lower length scale model parameters. Further, the method comprises estimating a plurality of transport properties based on the one or more initial material parameters and determining a mapping between a plurality of electrolyte compositions in a material database and associated plurality of transport properties using a lower length scale model based on the one or more lower length scale model parameters. The method further comprises computing a deviation index (DI) for each of the plurality of electrolyte compositions based on the associated plurality of transport properties and the estimated plurality of transport properties and selecting an electrolyte composition with minimum DI among the plurality of electrolyte compositions. Furthermore, the method comprises evaluating internal heat generation (Q) and capacity fade (C) of the selected electrolyte composition using a physics-based model based on the estimated plurality of transport properties and the one or more physics-based model parameters and optimizing an objective function based on the one or more optimization parameters to obtain revised material parameters. The objective function comprises a weighted sum of Q, C, and minimum DI. In an embodiment, the method is repeated until the revised material parameters satisfy a termination criteria.

In another aspect, a system for identifying electrolyte composition for optimal battery performance is provided. The system includes: a memory storing instructions; one or more communication interfaces; and one or more hardware processors coupled to the memory via the one or more communication interfaces, wherein the one or more hardware processors are configured by the instructions to: receive (i) one or more initial material parameters, (ii) one or more physics-based model parameters, (iii) one or more optimization parameters, and (iv) one or more lower length scale model parameters. Further the one or more hardware processors are configured to estimate a plurality of transport properties based on the one or more initial material parameters and determine a mapping between a plurality of electrolyte compositions in a material database and associated plurality of transport properties using a lower length scale model based on the one or more lower length scale model parameters. The one or more hardware processors are further configured to compute a deviation index (DI) for each of the plurality of electrolyte compositions based on the associated plurality of transport properties and the estimated plurality of transport properties and selecting an electrolyte composition with minimum DI among the plurality of electrolyte compositions. Furthermore, the one or more hardware processors are configured to evaluate internal heat generation (Q) and capacity fade (C) of the selected electrolyte composition using a physics-based model based on the estimated plurality of transport properties and the one or more physics-based model parameters and optimize an objective function based on the one or more optimization parameters to obtain revised material parameters. The objective function comprises a weighted sum of Q, C, and minimum DI. In an embodiment, the one or more hardware processors are configured to repeat the method until the revised material parameters satisfy a termination criteria.

In yet another aspect, there are provided one or more non-transitory machine-readable information storage mediums comprising one or more instructions which when executed by one or more hardware processors cause a method for identifying electrolyte composition for optimal battery performance. The method comprises receiving (i) one or more initial material parameters, (ii) one or more physics-based model parameters, (iii) one or more optimization parameters, and (iv) one or more lower length scale model parameters. Further, the method comprises estimating a plurality of transport properties based on the one or more initial material parameters and determining a mapping between a plurality of electrolyte compositions in a material database and associated plurality of transport properties using a lower length scale model based on the one or more lower length scale model parameters. The method further comprises computing a deviation index (DI) for each of the plurality of electrolyte compositions based on the associated plurality of transport properties and the estimated plurality of transport properties and selecting an electrolyte composition with minimum DI among the plurality of electrolyte compositions. Furthermore, the method comprises evaluating internal heat generation (Q) and capacity fade (C) of the selected electrolyte composition using a physics-based model based on the estimated plurality of transport properties and the one or more physics-based model parameters and optimizing an objective function based on the one or more optimization parameters to obtain revised material parameters. The objective function comprises a weighted sum of Q, C, and minimum DI. In an embodiment, the method is repeated until the revised material parameters satisfy a termination criteria.

It is to be understood that both the foregoing general description and the following detailed description are exemplary and explanatory only and are not restrictive of the invention, as claimed.

## BRIEF DESCRIPTION OF THE DRAWINGS

The accompanying drawings, which are incorporated in and constitute a part of this disclosure, illustrate exemplary embodiments and, together with the description, serve to explain the disclosed principles:

FIG. 1 illustrates an exemplary block diagram of a system for identifying electrolyte composition for optimal battery performance, according to some embodiments of the present disclosure.

FIG. 2 is a flow diagram illustrating method for identifying electrolyte composition for optimal battery performance, using the system of FIG. 1, according to some embodiments of the present disclosure.

FIG. 3 is a flow diagram illustrating modules of system of FIG. 1 used in implementing steps of method illustrated in FIG. 2, according to some embodiments of the present disclosure.

FIG. 4 illustrates the ionic conductivity as a function of salt concentration for various entries of a first materials database, according to some embodiments of the present disclosure.

FIG. 5 depicts the ionic conductivity versus concentration profiles of entries in a second materials database, according to some embodiments of the present disclosure.

## DETAILED DESCRIPTION OF EMBODIMENTS

Exemplary embodiments are described with reference to the accompanying drawings. In the figures, the left-most digit(s) of a reference number identifies the figure in which the reference number first appears. Wherever convenient, the same reference numbers are used throughout the drawings to refer to the same or like parts. While examples and features of disclosed principles are described herein, modifications, adaptations, and other implementations are possible without departing from the scope of the disclosed embodiments.

Electrolyte greatly influences battery safety and performance, and it is essential to select a suitable electrolyte while designing batteries. Electrolyte consists of salt, solvent, additives, etc. and each component influence the electrolyte properties and eventually battery performance. Existing methods of electrolyte design focus on optimizing the electrolyte composition on a stand-alone basis with respect to its properties. However, a holistic evaluation of the electrolyte is possible only after testing a battery made with the given electrolyte composition. Due to the challenge of extensive experimentation involved in fabricating and testing the battery, selecting an electrolyte for optimal battery performance is a slow process and is mostly based on trial-and-error. In order to address these challenges, embodiments of present disclosure provide a method and system for identifying electrolyte composition for optimal battery performance (both short term and long term performance). Initially a plurality of inputs are received from a user. The plurality of inputs comprise (i) one or more initial material parameters, (ii) one or more physics-based model parameters, (iii) one or more optimization parameters and (iv) one or more lower length scale model parameters. Next, a plurality of transport properties are estimated based on the initial material parameters. The plurality of transport properties comprise conductivity, diffusivity, and transference number. Then, a mapping between a plurality of electrolyte compositions in a material database and associated plurality of transport properties are determined using a lower length scale model based on the one or more lower length scale model parameters. Further, a deviation index is computed for each of the plurality of electrolyte compositions based on the associated plurality of transport properties and the estimated plurality of transport properties. Once the deviation index is computed for all the plurality of electrolyte compositions in the materials database, an electrolyte composition with minimum deviation index (DI) is selected. The selected electrolyte composition is closest match to desired electrolyte for the received user input. Once the minimum deviation index is determined and corresponding electrolyte is selected, internal heat generation (Q) and capacity fade (C) are evaluated based on the one or more physics-based model parameters, estimated transport properties and the selected electrolyte composition using a physics-based model. Further, an objective function is optimized based on the one or more optimization parameters to obtain revised material parameters. The objective function comprises weighted sum of Q, C and DI. If the revised material parameters does not satisfy a termination criteria, the method is repeated with the revised material parameters. Once the termination criteria is satisfied, final optimized electrolyte composition and its transport properties are given as output.

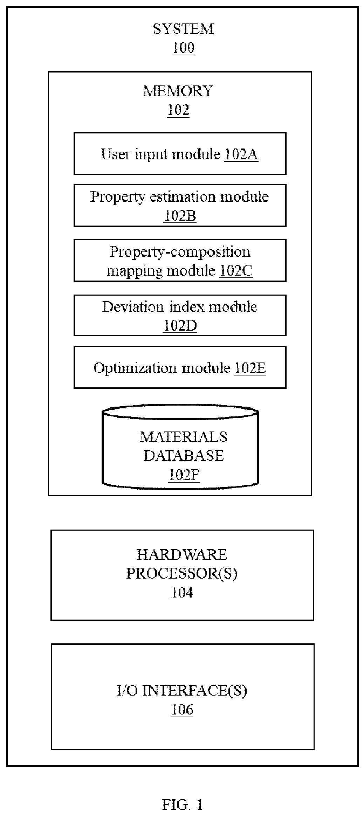

Referring now to the drawings, and more particularly to FIGS. 1 to 5, where similar reference characters denote corresponding features consistently throughout the figures, there are shown preferred embodiments and these embodiments are described in the context of the following exemplary system and/or method.

FIG. 1 illustrates an exemplary block diagram for identifying electrolyte composition for optimal battery performance. In an embodiment, the system 100 includes one or more processors (104), communication interface device(s) (106) or Input/Output (I/O) interface(s) (106) or user interface (106), and one or more data storage devices or memory (102) operatively coupled to the one or more processors (104). The memory (102) comprises a user input module (102A), a property estimation module (102B), a property-composition mapping module (102C), a deviation index module (102D), an optimization module (102E), and a materials database (102F). The one or more processors (104) that are hardware processors can be implemented as one or more microprocessors, microcomputers, microcontrollers, digital signal processors, central processing units, state machines, logic circuitries, and/or any devices that manipulate signals based on operational instructions. Among other capabilities, the processor(s) is configured to fetch and execute computer-readable instructions stored in the memory. In an embodiment, the system 100 can be implemented in a variety of computing systems, such as laptop computers, notebooks, hand-held devices, workstations, mainframe computers, servers, a network cloud, and the like.

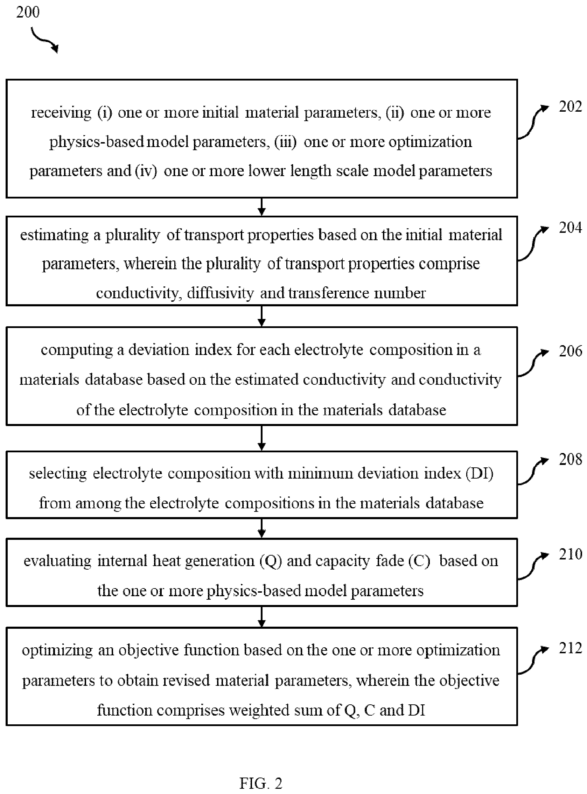

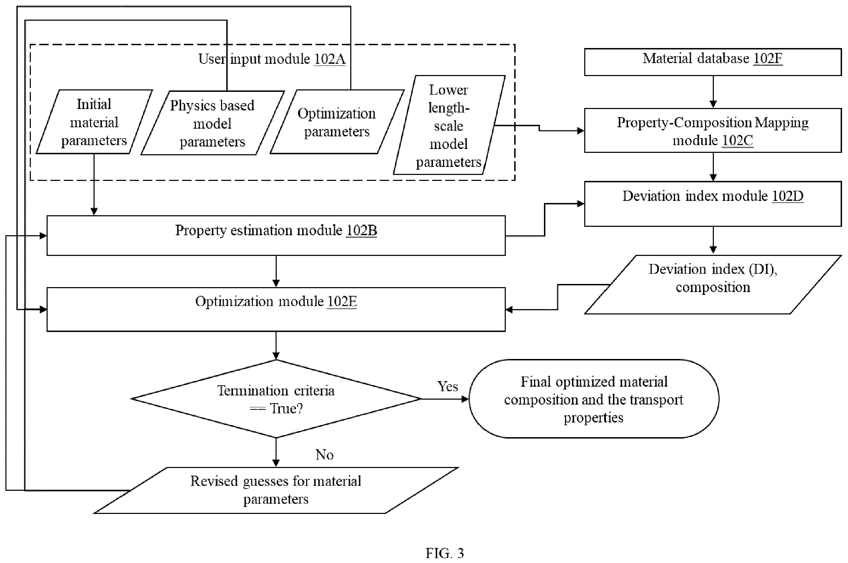

The I/O interface device(s) (106) can include a variety of software and hardware interfaces, for example, a web interface, a graphical user interface, and the like and can facilitate multiple communications within a wide variety of networks N/W and protocol types, including wired networks, for example, LAN, cable, etc., and wireless networks, such as WLAN, cellular, or satellite. In an embodiment, the I/O interface device(s) (106) receives a material as input and gives property of the material as output. The memory (102) may include any computer-readable medium known in the art including, for example, volatile memory, such as static random access memory (SRAM) and dynamic random access memory (DRAM), and/or non-volatile memory, such as read only memory (ROM), erasable programmable ROM, flash memories, hard disks, optical disks, and magnetic tapes. Functions of the components of system 100 are explained in conjunction with flow diagram depicted in FIGS. 2 and 3 for identifying electrolyte composition for optimal battery performance.

In an embodiment, the system 100 comprises one or more data storage devices or the memory (102) operatively coupled to the processor(s) (104) and is configured to store instructions for execution of steps of the method (200) depicted in FIG. 2 by the processor(s) or one or more hardware processors (104). The steps of the method of the present disclosure will now be explained with reference to the components or blocks of the system 100 as depicted in FIG. 1, the steps of flow diagram as depicted in FIGS. 2 and 3. Although process steps, method steps, techniques or the like may be described in a sequential order, such processes, methods, and techniques may be configured to work in alternate orders. In other words, any sequence or order of steps that may be described does not necessarily indicate a requirement that the steps to be performed in that order. The steps of processes described herein may be performed in any order practical. Further, some steps may be performed simultaneously.

FIG. 2 is a flow diagram illustrating method 200 for identifying electrolyte composition for optimal battery performance, according to some embodiments of the present disclosure. The steps of method 200 are executed by using components 102A-102F of system 100 as illustrated in FIG. 3. At step 202 of the method 200, the one or more hardware processors (104) are configured to receive user inputs via the user input module (102A). The user inputs comprise (i) one or more initial material parameters, (ii) one or more physics-based model parameters, (iii) one or more optimization parameters, and (iv) one or more lower length scale model parameters. In an embodiment, the one or more initial material parameters comprise initial guesses for electrolyte material parameters ($p_1$, $p_2$, $p_3$, ..., $p_{n_1}$, $q_1$, $q_2$, ..., $q_{n_2}$ and $r_1$, $r_2$, ...,$r_{n_3}$) provided by a user. In another embodiment, the one or more initial material parameters are revised material parameters obtained from the optimization module (102E). The one or more physics-based model parameters include the parameters required by the physics-based model to predict battery performance metrics like electrochemical performance, internal heat generation and capacity fade for given operating conditions. The one or more physics-based model parameters include electrode properties (for example, diffusivity of Lithium (Li) in the electrodes, electrode particle size, porosity), reaction kinetics properties (for example, Butler-Volmer properties for intercalation reaction, SEI/Li plating reaction parameters), operating conditions (for example, C-rate of charging/discharging, charging mode (CC/CC-CV), number of cycles of operation, cut off-voltage) and thermal parameters (for example, density, specific heat of battery, thermal conductivity and initial battery temperature).

The one or more optimization parameters include parameters needed for the optimization module (102E) to define the objective function ($k_1$, $k_2$, $k_3$) and constraints and run the optimization algorithm to estimate the revised guesses for material parameters. The optimization parameters include tolerance values on the electrolyte material parameters, and design space of electrolyte material parameters. The one or more lower length scale model parameters are used to estimate transport properties of the electrolyte. If the lower length scale model is based on Molecular Dynamics (MD) simulations, then the one or more lower length scale model parameters are force field parameters necessary to set up the MD simulation for the electrolyte. If the lower length scale model is an Artificial Intelligence (AI) or a Machine Learning (ML) model, then the one or more lower length scale model parameters refer to the neural-network or Support Vector Machine (SVM) parameters or similar such ML based model to estimate the transport properties. The lower length scale model can also include experimental data in some embodiments.

Once the user inputs are received, a plurality of transport properties are estimated at step 204 of the method 200 via the property estimation module (102B). The plurality of transport properties comprising conductivity ($\kappa_{input}(c_e, T)$), diffusivity ($D_{input}(c_e, T)$), and transference number ($t^+_{input}(c_e, T)$) are estimated based on the one or more initial material parameters. The transport properties are primarily a function of concentration ($c_e$) and temperature (T). In an embodiment, expressions comprising a plurality of fitting parameters for conductivity ($p_1$, $p_2$, $p_3$, ..., $p_{n_1}$), diffusivity ($q_1$, $q_2$, ..., $q_{n_2}$) and transference number ($r_1$, $r_2$, ..., $r_{n_3}$) are used. The expressions are applicable to a wide range of electrolytes. Equations 1 and 2 are example expressions for estimating conductivity, that have 9 and 6 fitting parameters respectively.

$$\kappa(c_e, T_{var}) = p_1 c_e (p_2 c_e + p_3 c_e^2 + (p_4 + p_5 c_e + p_6 c_e^2)T_{var} + p7(p_8 + p_9 c_e)T_{var}^2)^2 ....(1)$$

$$\kappa(c_e, T_{var}) = p_1(1 + (T_{var} - p_2))c_e \frac{(1+p_3\sqrt{c_e}+p_4(1+p_5 \exp\left(\frac{1000}{T_{var}}\right))c_e}{1+c_e^4 p_6 \exp\left(\frac{1000}{T_{var}}\right)} ...(2)$$

Once the transport properties are estimated, at step 206 of the method 200, a mapping between a plurality of electrolyte compositions in the material database 102F and associated plurality of transport properties is obtained using a lower length scale model based on the one or more lower length scale model parameters, via the property-composition mapping module (102C). In alternate embodiments, the property-composition mapping is determined before performing the step 204 or in parallel to execution of step 204. The lower length scale model can be either based on experimental data or Molecular Dynamics (MD) simulations or Density Functional Theory (DFT) simulations or Artificial Intelligence (AI) based model or Machine Learning (ML) based model.

Once the transport properties are estimated and property-composition mapping of all electrolyte compositions in the materials database is determined, a deviation index is computed for each electrolyte composition (with index $i$) in the materials database (102F) at step 208 of the method 200 using the deviation index module (102D). The deviation index is computed according to equation 3 based on the conductivity estimated in the step 204 via the property estimation module (102B) and conductivity of each electrolyte composition in the materials database determined in the step 206 by the property-composition mapping module (102C). In equation 3, square of difference of conductivity of electrolyte composition in the material database ($\kappa_i(c_e, T_0)$) and estimated conductivity ($\kappa_{input}(c_e, T_0)$) integrated over a range of concentrations ($c_0$ to $c_{max}$) is divided by square of sum of conductivity of electrolyte composition in the material database and estimated conductivity integrated over a range of concentrations to compute the deviation index.

$$DI\_vec[i] = \frac{\int_{c_0}^{c_{max}}\left(\kappa_i(c_e, T_0) - \kappa_{input}(c_e, T_0)\right)^2 dc_e}{\int_{c_0}^{c_{max}}\left(\kappa_i(c_e, T_0) + \kappa_{input}(c_e, T_0)\right)^2 dc_e} \qquad ....(3)$$

In equation 3, *DI_vec[i]* is the deviation index of the electrolyte composition with index *i* in the materials database (102F), $\kappa_i$ is conductivity of the electrolyte composition with index *i* in the materials database (102F), $\kappa_{input}$ is the estimated conductivity, $c_e$ is a concentration of electrolyte among range of concentrations $c_0$ to $c_{max}$ and $T_0 = 298$ *K* is the nominal operating temperature of the battery.

Once the deviation index is calculated for all the electrolyte compositions in the materials database, an electrolyte composition with minimum deviation index (DI) is selected at step 210 of the method 200. The selected electrolyte composition and minimum deviation index (DI) are given as output of deviation index module (102D) which are further provided as input to the optimization module (102E). The steps 212 and 214 of the method 200 are implemented via the optimization module (102E). At the step 212, battery performance parameters including internal heat generation (Q) and capacity fade (C) of a battery are estimated based on the transport properties estimated at the step 204, one or more physics-based model parameters and the selected electrolyte composition using a physics-based model. In case the physics-based model is computationally expensive, the optimization module incorporates a surrogate model. This surrogate model aids in efficiently exploring the solution space, reducing computational expenses while still maintaining accurate optimization results. By leveraging statistical techniques, the surrogate model guides the selection of candidate points for evaluation, reducing reliance on expensive physics-based models. This balance between computational efficiency and accurate estimation enhances the overall effectiveness of the optimization module.

Further, at the step 214, an objective function is optimized based on the one or more optimization parameters to obtain revised material parameters. The objective function comprises weighted sum of Q, C and DI according to equation 4.

$$F = k_1 Q + k_2 C + k_3 DI \qquad ....(4)$$

In equation 4, F denotes the objective function to be optimized, $k_1$, $k_2$, $k_3$ are the optimization parameters, Q is the internal heat generation, C is the capacity fade and DI is the minimum deviation index. To optimize the objective function, various global optimization techniques such as Genetic Algorithm, Particle Swarm Optimization, Bayesian Optimization, or Simulated Annealing can be employed.

If the revised material parameters do not satisfy a termination criteria, the steps of the method 200 are repeated with the revised material parameters. In an embodiment, difference in electrolyte parameter values (i.e., transport properties of the electrolyte) between consecutive iterations is computed and if this difference falls below a predefined tolerance value, then termination criteria is said to be satisfied. In another embodiment, number of iterations the user wants to run the method 200 for is received as one of the optimization parameter from the user. Then, execution of the method 200 for the specified number of iterations is considered as the termination criteria. Once the termination criteria is satisfied, final optimized electrolyte composition and its transport properties are given as output.

By seamlessly integrating the optimization module within the battery framework, embodiments of present disclosure identify most suitable battery electrolyte composition. Through iterative refinement based on the objective function, the optimization module significantly contributes to enhancing battery performance. The deviation index is utilized to estimate the similarity between the estimated electrolyte ionic conductivity, evaluated based on the parameters obtained from the optimization module, and the ionic conductivity of the electrolyte for the entries stored in the materials database. While an exact match may not always be attainable, the optimization module intelligently selects the entry with the minimum deviation index because of its inclusion in the objective function. Incorporation of the deviation index value into the objective function serves to expedite the search algorithm, facilitating efficient and accelerated convergence towards an optimal solution.

## CASE STUDIES

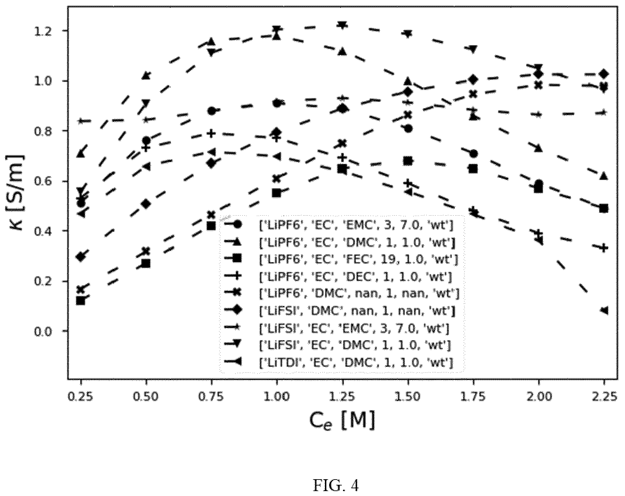

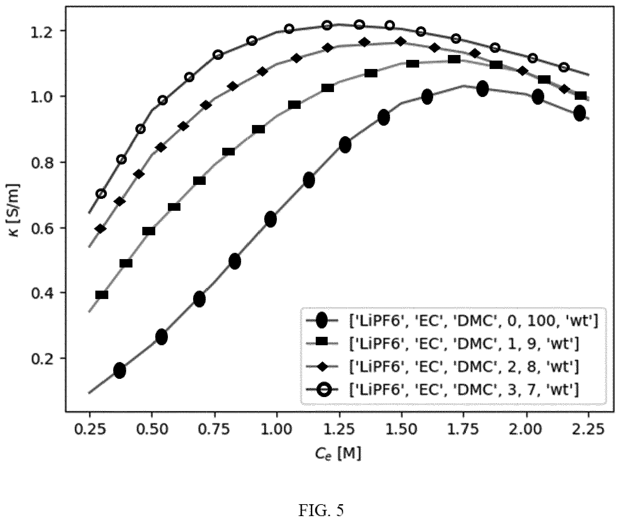

For the case studies, 2 materials databases are considered. In the first materials database, there are 9 different electrolyte compositions of 3 different salts and solvents of different compositions. The second materials database has LiPF6 in 4 different concentration ratios of EC:DMC (Ethylene carbonate: Dimethyl carbonate). FIG. 4 illustrates the ionic conductivity as a function of salt concentration for various entries of a first material database which consists of three different kinds of salts: LiPF6, LiFSI and LiTDI. The solvents considered are EC:DMC, pure DMC, EC:FEC, EC:EMC. FIG. 5 depicts the ionic conductivity vs concentration profiles of entries in the second materials database. This database consists of entries with different composition of solvent EC:DMC for same salt LiPF6. The physics-based model parameters considered are listed in Tables 1,2,3.

Table 1

| Parameter | Units | Description | value |
|---|---|---|---|
| $C_{max,n}$ | $kmol/m^3$ | Max concentration of anode | 31.1 |
| $C_{max,p}$ | $kmol/m^3$ | Max concentration of cathode | 49.52 |
| $C_2^n{}_2$ | $kmol/m^3$ | Average Electrolyte concentration | 1 |
| $\delta_n$ | $\mu m$ | length of anode | 48.7 |
| $\delta_p$ | $\mu m$ | length of cathode | 40.75 |
| $\delta_s$ | $\mu m$ | length of separator | 25 |
| $C_{1,n}^0$ | $kmol/m^3$ | initial concentration of anode | 1.4 |
| $C_{1,p}^0$ | $kmol/m^3$ | initial concentration of cathode | 46.55 |
| $\alpha$ | - | transfer coefficient | 0.5 |
| $R_n$ | $\mu m$ | radius of anode particle | 10 |
| $R_p$ | $\mu m$ | radius of cathode particle | 5 |
| $Tref$ | $K$ | Reference temperature | 298.15 |
| $E_{e,n}$ | - | Electrolyte phase volume fraction in anode | 0.32 |
| $E_{e,s}$ | - | Electrolyte phase volume fraction in separator | 0.4 |
| $E_{e,p}$ | - | Electrolyte phase volume fraction in cathode | 0.33 |
| $brugg$ | - | Bruggeman coefficient | 2.2 |
| $k_{0,n}$ | $\dfrac{A/m^2}{(\frac{mol}{m^3})^{1.5}}$ | Rate constant of intercalation reaction in negative electrode | $3\times10^{-6}$ |
| $k_{0,p}$ | $\dfrac{A/m^2}{(\frac{mol}{m^3})^{1.5}}$ | Rate constant of intercalation reaction in positive electrode | $3\times10^{-6}$ |
| $E_{k,n}^a$ | $J/mol$ | Activation energy of intercalation reaction in negative electrode | $68\times10^3$ |
| $E_{k,p}^a$ | $J/mol$ | Activation energy of intercalation reaction in positive electrode | $40\times10^3$ |
| $E_{f,n}$ | - | Filler fraction in negative electrode | 0.0194 |
| $E_{f,p}$ | - | Filler fraction in positive electrode | 0.0536 |
| $Area_n$ | $m^2$ | Total coated area of negative electrode | $6048.8\text{x}1\,0^{-4}$ |
| $Area_p$ | $m^2$ | Total coated area of positive electrode | $6304.3\text{x}1\,0^{-4}$ |
| $D_{0,n}$ | m2 /s | Diffusivity of negative electrode | $3\times10^{-14}$ |

(continued)

| Parameter | Units | Description | value |
|---|---|---|---|
| $D_{0,p}$ | m2 /s | Diffusivity of positive electrode | $3 \times 10^{-14}$ |
| $E_{D,n}^{a'}$ | J/mol | Activation energy of solid phase diffusion in negative electrode | $30 \times 10^3$ |
| $E_{D,p}^{a}$ | J/mol | Activation energy of solid phase diffusion in positive electrode | $30 \times 10^3$ |
| $\sigma_n$ | S/m | Conductivity in negative electrode | 100 |
| $\sigma_p$ | S/m | Conductivity in positive electrode | 3.8 |

The physics based model further comprises of electrochemical, thermal, and ageing models. The one or more physics based model parameters include parameters related to the electrochemical, thermal, and ageing models. Parameters of thermal and ageing models are listed in tables 2 and 3 respectively. The electrochemical model accepts one or more parameters listed in table 1. The optimization parameters considered in case studies are listed in table 4.

Table 2

| Parameter | Units | Description | Value |
|---|---|---|---|
| $C_p$ | J/Kg.K | Cell specific heat | 1000 |
| $h_{convection}$ | W/m$^2$.K | Convective heat transfer coefficient | 25 |
| $T_{ambient}$ | K | Ambient temperature | 298.15 |
| $m$ | Kg | Mass of the cell | $202.42 \times 10^{-3}$ |
| $Area_{cell}$ | $m^2$ | Cell surface area | $229.85 \times 10^{-4}$ |
| $V_{cell}$ | $m^3$ | Cell volume | $82.88 \times 10^{-6}$ |

Table 3

| Paramet er | Units | Description | Value |
|---|---|---|---|
| $k_{0,SEI}$ | m/s | Kinetic rate constant of SEI reaction | $1 \times 10^{-12}$ |
| $E_{k,SEI}^{a}$ | J/mol | Activation energy of SEI reaction | 30000 |
| $c_{EC}^{0}$ | mol /m$^3$ | Concentration of Ethyl Carbonate(EC) in bulk electrolyte | $4.541 \times 10^3$ |
| $D_{0,EC}$ | m$^2$/s | Diffusivity of EC | $2 \times 10^{-18}$ |
| $E_{D,EC}^{a}$ | J/mol | Activation energy of EC diffusion reaction | 30000 |
| $i_{0,Li}$ | A/m$^2$ | Exchange current density of plating reaction | 0.001 |
| $E_{k,plating}^{a}$ | J/mol | Activation energy of plating reaction | 68000 |
| $M_{SEI}$ | Kg /mol | Molar weight of SEI | 0.162 |
| $\rho_{SEI}$ | Kg /m$^3$ | Density of SEI | 1690 |
| $\kappa_{SEI}$ | S/m | Conductivity of SEI layer | $5 \times 10^{-6}$ |
| $M_{Li}$ | Kg /mol | Molar weight of Lithium metal | $6.94 \times 10^{-3}$ |

(continued)

| Paramet er | Units | Description | Value |
|---|---|---|---|
| $\rho_{Li}$ | $Kg\,/m^3$ | Density of Lithium metal | 534 |

Table 4

| Optimization Algorithm : Bayesian optimization | |
|---|---|
| Parameter | Value |
| Acquisition function | Expected Improvement |
| Acquisition runs | 5 |
| Total iterations | 30 |
| Batch size | 5 |
| k1, k2, k3 (prefactors of objective function) | Set based on case study |

CASE STUDY 1: In this case study goal is to find the optimized electrolyte with better cycle life and low temperature rise from the first materials database. C-rate is taken as 6C, and ambient temperature is 298.15K. The value of $k_1$, $k_2$, $k_3$ is set as 1 in Equation 4. Initial configuration/guesses given by user include $LiPF_6$ salt in EC:FEC 19:1 wt.%. Cycle life for this initial configuration was calculated as 696 and maximum temperature was 56°C. After execution of the method 200, the optimal material identified was LiFSi in EC:DMC 1:1 wt.%. This gave a cycle life of 1694 and maximum temperature was 50°C with a deviation index of 0.036. Thus, by applying the method 200 for 30 iterations, cycle life of the battery was improved by 143% and temperature decreased by 6°C. It is to be noted that the method 200 optimizes capacity fade and internal heat generation which are directly correlated with the cycle life and maximum temperature, respectively.

CASE STUDY 2: In this case study goal is to find an optimal electrolyte with lower temperature rise from the first materials database. Cycle life was ignored in this case study. C-rate is taken as 6C, and temperature is 298.15K. The value of $k_1$, $k_3$ is set as 1, and $k_2$ as 0 in Equation 4. Initial configuration/guesses given by user included $LiPF_6$ salt in EC:FEC 19:1 wt.%. Maximum temperature was calculated as 52.7°C. After execution of the method 200 for 30 iterations, the optimal material identified was LiFSi in DMC. This resulted in maximum temperature 48.9°C with a deviation index of 0.000781. Thus, by applying the method 200, temperature decreased by 3.8°C.

CASE STUDY 3: In this case study goal is to find an optimal electrolyte from the first materials database with improved cycle life. Temperature rise was ignored in this case study. The value of $k_1$, $k_3$ are set as 1 and $k_1$ as 0 in Equation 4. C-rate is taken as 6C, and temperature is 298.15K. Initial configuration/guesses given by user included $LiPF_6$ salt in EC:FEC 19:1 wt.%. Cycle life was calculated as 696. After execution of the method 200 for 30 iterations, the optimal material identified was LiFSi in EC:DMC 1.1 wt.%. This resulted in cycle life of 1710 with a deviation index of 0.026. Thus, the method 200 improved cycle life of the battery by 146%.

CASE STUDY 4: In this case study goal is to find an optimal concentration of an electrolyte from the second materials database which has improved cycle life and reduced temperature rise. The value of $k_1$, $k_2$, $k_3$ is set as 1 in Equation 4. C-rate is taken as 6C, and temperature is 298.15K. Initial configuration/guesses given by user included $LiPF_6$ salt in DMC 100 wt.%. Cycle life was calculated as 593 and maximum temperature was 54.5°C. Execution of the method 200 for 30 iterations resulted in the optimal concentration of $LiPF_6$ salt with EC:DMC in the ratio 3:7. This resulted in cycle life of 1657, maximum temperature of 50.6°C with a deviation index of 0.00064. Thus, the method 200 improved cycle life of the battery by 179.4% and temperature decrease of 3.9°C.

CASE STUDY 5: In this case study goal is to find an optimal concentration of an electrolyte from the second materials database which has improved cycle life and reduced temperature rise. C-rate is taken as 6C, and temperature is 298.15K. The value of $k_1$, $k_2$, $k_3$ is set as 1 in Equation 4. Initial configuration/guesses given by user included $LiPF_6$ salt in EC:DMC 1:9 wt.%. Cycle life was calculated as 1419 and maximum temperature was 51.8°C. Execution of the method 200 for 30 iterations resulted in the optimal concentration of $LiPF_6$ salt with EC:DMC in the ratio 3:7. This resulted in cycle life of 1657, maximum temperature of 50.6°C with a deviation index of 0.00064. Thus, the method 200 improved cycle life of the battery by 17% and temperature decrease of 1.16°C.

The case studies illustrate that method 200 is capable of identifying a new electrolyte from the material database as well as identify optimal electrolyte composition of a given salt and solvent ratios which results in better performance of the battery.

The written description describes the subject matter herein to enable any person skilled in the art to make and use the embodiments. The scope of the subject matter embodiments is defined by the claims and may include other modifications that occur to those skilled in the art. Such other modifications are intended to be within the scope of the claims if they have similar elements that do not differ from the literal language of the claims or if they include equivalent elements with insubstantial differences from the literal language of the claims.

It is to be understood that the scope of the protection is extended to such a program and in addition to a computer-readable means having a message therein; such computer-readable storage means contain program-code means for implementation of one or more steps of the method, when the program runs on a server or mobile device or any suitable programmable device. The hardware device can be any kind of device which can be programmed including e.g., any kind of computer like a server or a personal computer, or the like, or any combination thereof. The device may also include means which could be e.g., hardware means like e.g., an applicationspecific integrated circuit (ASIC), a field-programmable gate array (FPGA), or a combination of hardware and software means, e.g., an ASIC and an FPGA, or at least one microprocessor and at least one memory with software processing components located therein. Thus, the means can include both hardware means, and software means. The method embodiments described herein could be implemented in hardware and software. The device may also include software means. Alternatively, the embodiments may be implemented on different hardware devices, e.g., using a plurality of CPUs.

The embodiments herein can comprise hardware and software elements. The embodiments that are implemented in software include but are not limited to, firmware, resident software, microcode, etc. The functions performed by various components described herein may be implemented in other components or combinations of other components. For the purposes of this description, a computerusable or computer readable medium can be any apparatus that can comprise, store, communicate, propagate, or transport the program for use by or in connection with the instruction execution system, apparatus, or device.

The illustrated steps are set out to explain the exemplary embodiments shown, and it should be anticipated that ongoing technological development will change the manner in which particular functions are performed. These examples are presented herein for purposes of illustration, and not limitation. Further, the boundaries of the functional building blocks have been arbitrarily defined herein for the convenience of the description. Alternative boundaries can be defined so long as the specified functions and relationships thereof are appropriately performed. Alternatives (including equivalents, extensions, variations, deviations, etc., of those described herein) will be apparent to persons skilled in the relevant art(s) based on the teachings contained herein. Such alternatives fall within the scope of the disclosed embodiments. Also, the words "comprising," "having," "containing," and "including," and other similar forms are intended to be equivalent in meaning and be open ended in that an item or items following any one of these words is not meant to be an exhaustive listing of such item or items or meant to be limited to only the listed item or items. It must also be noted that as used herein and in the appended claims, the singular forms "a," "an," and "the" include plural references unless the context clearly dictates otherwise.

Furthermore, one or more computer-readable storage media may be utilized in implementing embodiments consistent with the present disclosure. A computer-readable storage medium refers to any type of physical memory on which information or data readable by a processor may be stored. Thus, a computer-readable storage medium may store instructions for execution by one or more processors, including instructions for causing the processor(s) to perform steps or stages consistent with the embodiments described herein. The term "computer-readable medium" should be understood to include tangible items and exclude carrier waves and transient signals, i.e., be non-transitory. Examples include random access memory (RAM), read-only memory (ROM), volatile memory, nonvolatile memory, hard drives, CD ROMs, DVDs, flash drives, disks, and any other known physical storage media.

It is intended that the disclosure and examples be considered as exemplary only, with a true scope of disclosed embodiments being indicated by the following claims.

**Claims**

1. A processor implemented method (200), comprising:

receiving (202), via one or more hardware processors, i) one or more initial material parameters, ii) one or more physics-based model parameters, iii) one or more optimization parameters, and iv) one or more lower length scale model parameters;

estimating (204), via the one or more hardware processors, a plurality of transport properties based on the one or more initial material parameters;

determining (206), via the one or more hardware processors, a mapping between a plurality of electrolyte compositions in a material database and associated plurality of transport properties using a lower length scale model based on the one or more lower length scale model parameters;

computing (208), via the one or more hardware processors, a deviation index (DI) for each of the plurality of electrolyte compositions based on the associated plurality of transport properties and the estimated plurality of transport properties;

selecting (210), via the one or more hardware processors, an electrolyte composition with minimum DI among the plurality of electrolyte compositions;

evaluating (212), via the one or more hardware processors, internal heat generation 'Q' and capacity fade 'C' of the selected electrolyte composition using a physics-based model based on the estimated plurality of transport properties and the one or more physics-based model parameters; and

optimizing (214), via the one or more hardware processors, an objective function based on the one or more optimization parameters to obtain revised material parameters, wherein the objective function comprises a weighted sum of Q, C, and minimum DI.

2. The method as claimed in claim 1, wherein the one or more initial material parameters are information on one or more parameters related to electrode and electrolyte of a battery, provided by a user, and wherein the one or more physics-based model parameters comprise electrode properties, reaction kinetics properties, operating conditions of the battery, number of cycles of operation, cut off-voltage, and thermal parameters.

3. The method as claimed in claim 1, wherein the one or more optimization parameters comprise tolerance values on the objective function, design space of electrolyte material parameters, and lower and upper limits for the electrolyte material properties.

4. The method as claimed in claim 1, wherein the plurality of transport properties comprise conductivity, diffusivity, and transference number.

5. The method as claimed in claim 1, wherein the deviation index for each of the plurality of electrolyte compositions

$$DI[i] = \frac{\int_{c_0}^{c_{max}}\left(\kappa_i\left(c_e, T_0\right) - \kappa_{input}\left(c_e, T_0\right)\right)^2 dc_e}{\int_{c_0}^{c_{max}}\left(\kappa_i\left(c_e, T_0\right) + \kappa_{input}\left(c_e, T_0\right)\right)^2 dc_e}$$

is calculated by the equation:                     , wherein $\kappa_i(c_e, T_0)$ is conductivity of the ith electrolyte composition among the plurality of electrolyte compositions, $\kappa_{input}(c_e, T_0)$ is the estimated conductivity, and $c_0$ to $c_{max}$ is a range of concentrations of the electrolyte.

6. A system (100), comprising:

a memory (102) storing instructions;
one or more Input/Output 'I/O' interfaces (106); and
one or more hardware processors (104) coupled to the memory (102) via the one or more communication interfaces (106), wherein the one or more hardware processors (104) are configured by the instructions to:

receive i) one or more initial material parameters, ii) one or more physics-based model parameters, iii) one or more optimization parameters, and iv) one or more lower length scale model parameters;

estimating a plurality of transport properties based on the one or more initial material parameters;

determining a mapping between a plurality of electrolyte compositions in a material database and associated plurality of transport properties using a lower length scale model based on the one or more lower length scale model parameters;

computing a deviation index 'DI' for each of the plurality of electrolyte compositions based on the associated plurality of transport properties and the estimated plurality of transport properties;

selecting an electrolyte composition with minimum DI among the plurality of electrolyte compositions;

evaluating internal heat generation 'Q' and capacity fade 'C' of the selected electrolyte composition using a physics-based model based on the estimated plurality of transport properties and the one or more physics-based model parameters; and

optimizing an objective function based on the one or more optimization parameters to obtain revised material parameters, wherein the objective function comprises weighted sum of Q, C, and minimum DI.

7. The system as claimed in claim 6, wherein the one or more initial material parameters are information on one or more parameters related to electrode and electrolyte of a battery, provided by a user, and wherein the one or more physics-based model parameters comprise electrode properties, reaction kinetics properties, operating conditions of the battery, number of cycles of operation, cut off-voltage, and thermal parameters.

8. The system as claimed in claim 6, wherein the one or more optimization parameters comprise tolerance values on the objective function, design space of electrolyte material parameters, and lower and upper limits for the electrolyte material properties.

9. The system as claimed in claim 6, wherein the plurality of transport properties comprise conductivity, diffusivity, and transference number.

10. The system as claimed in claim 6, wherein the deviation index for each of the plurality of electrolyte compositions

$$DI[i] = \frac{\int_{c_0}^{c_{max}}\left(\kappa_i(c_e,T_0)-\kappa_{input}(c_e,T_0)\right)^2 dc_e}{\int_{c_0}^{c_{max}}\left(\kappa_i(c_e,T_0)+\kappa_{input}(c_e,T_0)\right)^2 dc_e}$$

is calculated by the equation: , wherein $\kappa_i(c_e,T_0)$ is conductivity of the ith electrolyte composition among the plurality of electrolyte compositions, $\kappa_{input}(c_e,T_0)$ is the estimated conductivity, and $c_0$ to $c_{max}$ is a range of concentrations of the electrolyte.

11. One or more non-transitory machine-readable information storage mediums comprising one or more instructions which when executed by one or more hardware processors cause:

    receiving i) one or more initial material parameters, ii) one or more physics-based model parameters, iii) one or more optimization parameters, and iv) one or more lower length scale model parameters;
    estimating a plurality of transport properties based on the one or more initial material parameters;
    determining a mapping between a plurality of electrolyte compositions in a material database and associated plurality of transport properties using a lower length scale model based on the one or more lower length scale model parameters;
    computing a deviation index 'DI' for each of the plurality of electrolyte compositions based on the associated plurality of transport properties and the estimated plurality of transport properties;
    selecting an electrolyte composition with minimum DI among the plurality of electrolyte compositions;
    evaluating internal heat generation 'Q' and capacity fade 'C' of the selected electrolyte composition using a physics-based model based on the estimated plurality of transport properties and the one or more physics-based model parameters; and
    optimizing an objective function based on the one or more optimization parameters to obtain revised material parameters, wherein the objective function comprises a weighted sum of Q, C, and minimum DI.

12. The one or more non-transitory machine-readable information storage mediums of claim 11, wherein the one or more initial material parameters are information on one or more parameters related to electrode and electrolyte of a battery, provided by a user, and wherein the one or more physics-based model parameters comprise electrode properties, reaction kinetics properties, operating conditions of the battery, number of cycles of operation, cut off-voltage, and thermal parameters.

13. The one or more non-transitory machine-readable information storage mediums of claim 11, wherein the one or more optimization parameters comprise tolerance values on the objective function, design space of electrolyte material parameters, and lower and upper limits for the electrolyte material properties.

14. The one or more non-transitory machine-readable information storage mediums of claim 11, wherein the plurality of transport properties comprise conductivity, diffusivity, and transference number.

15. The one or more non-transitory machine-readable information storage mediums of claim 11, wherein the deviation index for each of the plurality of electrolyte compositions is calculated by the equation:

$$DI[i] = \frac{\int_{c_0}^{c_{max}}\left(\kappa_i(c_e,T_0)-\kappa_{input}(c_e,T_0)\right)^2 dc_e}{\int_{c_0}^{c_{max}}\left(\kappa_i(c_e,T_0)+\kappa_{input}(c_e,T_0)\right)^2 dc_e}$$

, wherein $\kappa_i(c_e,T_0)$ is conductivity of the ith electrolyte composition among the plurality of electrolyte compositions, $\kappa_{input}(c_e,T_0)$ is the estimated conductivity, and $c_0$ to $c_{max}$ is a range

of concentrations of the electrolyte.

SYSTEM
100

MEMORY
102

User input module 102A

Property estimation module
102B

Property-composition
mapping module 102C

Deviation index module
102D

Optimization module 102E

MATERIALS
DATABASE
102F

HARDWARE
PROCESSOR(S)
104

I/O INTERFACE(S)
106

FIG. 1

200

receiving (i) one or more initial material parameters, (ii) one or more physics-based model parameters, (iii) one or more optimization parameters and (iv) one or more lower length scale model parameters ⟳ 202

estimating a plurality of transport properties based on the initial material parameters, wherein the plurality of transport properties comprise conductivity, diffusivity and transference number ⟳ 204

computing a deviation index for each electrolyte composition in a materials database based on the estimated conductivity and conductivity of the electrolyte composition in the materials database ⟳ 206

selecting electrolyte composition with minimum deviation index (DI) from among the electrolyte compositions in the materials database ⟳ 208

evaluating internal heat generation (Q) and capacity fade (C) based on the one or more physics-based model parameters ⟳ 210

optimizing an objective function based on the one or more optimization parameters to obtain revised material parameters, wherein the objective function comprises weighted sum of Q, C and DI ⟳ 212

FIG. 2

Material database 102F

Property-Composition Mapping module 102C

Deviation index module 102D

Deviation index (DI), composition

User input module 102A

Initial material parameters

Physics based model parameters

Optimization parameters

Lower length-scale model parameters

Property estimation module 102B

Optimization module 102E

Termination criteria == True?

Yes → Final optimized material composition and the transport properties

No → Revised guesses for material parameters

FIG. 3

FIG. 4

FIG. 5

Europäisches
Patentamt
European
Patent Office
Office européen
des brevets

# EUROPEAN SEARCH REPORT

**Application Number**

EP 23 18 1741

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | EP 3 719 909 A1 (TATA CONSULTANCY SERVICES LTD [IN]) 7 October 2020 (2020-10-07) * paragraphs [0002], [0005], [0006], [0029], [0031], [0036] – [0039], [0045], [0046] * * figure 2a * | 1-15 | INV.<br>G06F30/20<br>G06F30/25<br>G06F30/28<br>H01M10/056<br>G16C10/00<br>G16C20/20<br>G16C20/30 |
| X | CN 114 566 226 A (GUIZHOU MELINGLING POWER CO LTD) 31 May 2022 (2022-05-31) * paragraphs [0001], [0026], [0051] – [0054], [0063] – [0121] * | 1-15 | |
| X | CN 114 255 826 A (UNIV QINGHUA) 29 March 2022 (2022-03-29) * paragraphs [0001], [0005], [0052] – [0184] * * figure 1 * | 1-15 | |

**TECHNICAL FIELDS SEARCHED (IPC)**

G06F
H01M
G16C

The present search report has been drawn up for all claims

| Place of search | Date of completion of the search | Examiner |
|---|---|---|
| The Hague | 11 December 2023 | Grave, Christian |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
............................................................................
& : member of the same patent family, corresponding document

EPO FORM 1503 03.82 (P04C01)

**ANNEX TO THE EUROPEAN SEARCH REPORT
ON EUROPEAN PATENT APPLICATION NO.**

EP 23 18 1741

This annex lists the patent family members relating to the patent documents cited in the above-mentioned European search report.
The members are as contained in the European Patent Office EDP file on
The European Patent Office is in no way liable for these particulars which are merely given for the purpose of information.

11-12-2023

| Patent document cited in search report | | Publication date | Patent family member(s) | | Publication date |
|---|---|---|---|---|---|
| EP 3719909 | A1 | 07-10-2020 | EP | 3719909 A1 | 07-10-2020 |
| | | | US | 2020321080 A1 | 08-10-2020 |
| CN 114566226 | A | 31-05-2022 | NONE | | |
| CN 114255826 | A | 29-03-2022 | CN | 114255826 A | 29-03-2022 |
| | | | WO | 2023103150 A1 | 15-06-2023 |

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- IN 202221037132